(19) Europäisches Patentamt
European Patent Office
Office européen des brevets

(11) **EP 3 021 117 B1**

(12) **EUROPÄISCHE PATENTSCHRIFT**

(45) Veröffentlichungstag und Bekanntmachung des Hinweises auf die Patenterteilung:
**08.01.2020  Patentblatt 2020/02**

(51) Int Cl.:
*G01N 33/22* *(2006.01)*          *G01N 25/18* *(2006.01)*
*G01N 29/024* *(2006.01)*

(21) Anmeldenummer: **15003229.0**

(22) Anmeldetag: **12.11.2015**

(54) **VERFAHREN UND MESSVORRICHTUNG ZUR BESTIMMUNG VON SPEZIFISCHEN GRÖSSEN FÜR DIE GASBESCHAFFENHEIT**

METHOD AND MEASURING DEVICE FOR THE DETERMINATION OF SPECIFIC VALUES FOR THE CONSTITUTION OF GAS

PROCEDE ET DISPOSITIF DE MESURE DESTINES A LA DETERMINATION DE GRANDEURS SPECIFIQUES POUR LA CONSTITUTION DU GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.11.2014  EP 14003855**

(43) Veröffentlichungstag der Anmeldung:
**18.05.2016  Patentblatt 2016/20**

(73) Patentinhaber: **MEMS AG**
**5200 BRUGG (CH)**

(72) Erfinder: **Prêtre, Philippe**
**5405 Dättwil (CH)**

(74) Vertreter: **Steiner, Peter**
**Patente + Marken**
**Untere Wolfackerstrasse 16**
**8280 Kreuzlingen (CH)**

(56) Entgegenhaltungen:
EP-A1- 0 715 169          EP-A1- 2 015 056
EP-A1- 2 574 918          WO-A1-02/40992
WO-A1-2004/008136

**Beschreibung**

Die Erfindung bezieht sich auf ein Verfahren und auf eine Messvorrichtung zur Bestimmung von spezifischen Grössen für die Gasbeschaffenheit und/oder des Energieverbrauchs im Haushalt- und Industriebereich.

Die (Erd-)Gaszusammensetzung und damit die Gasbeschaffenheit wird künftig aufgrund von neuen Herkunftsquellen (Biogas, verflüssigtes Erdgas aus allen Weltgegenden, Wasserstoff aus der Verwertung von überschüssigem Strom bei der alternativen Energiegewinnung) mehr und häufiger schwanken als heute und damit in Gasanwendungsprozessen unterschiedliche, mitunter auch negative Auswirkungen haben. Mit der direkten Messung von spezifischen Grössen für die Gasbeschaffenheit vor Ort könnten die Prozesse auf die wechselnde Gasqualität eingestellt werden, um einen optimalen und sicheren Betrieb zu gewährleisten. Zu den spezifischen Grössen für die Gasbeschaffenheit gehören z.B. der Wobbe Index für Brennersteuerungen, die Luftzahl bei Energiegewinnungsanlagen (Industrieöfen, Brennstoffzellen, etc.), die Methanzahl für Gasmotoren oder der Brennwert zur Abrechnung der bezogenen Energiemenge. Letzteres setzt allerdings voraus, dass auch die bezogene Gasmenge gemessen wird, was heute bis auf wenige Ausnahmen mittels einer Volumendurchflussmessung mit Balgengaszählern (Haushalt) oder, bei Gasbezügern grosser Gasvolumina (Industrie), mittels Drehkolben-, Turbinenrad- oder Ultraschallzählern geschieht. All diese Messmittel sind nur zur Bestimmung des Betriebsvolumens geeignet. Um aus diesen Daten auf die bezogene, abrechenbare Energie zu schliessen, ist sowohl eine Umrechnung auf Normvolumen als auch die Kenntnis des Brennwerts des jeweils gelieferten Gases notwendig. Beides geschieht nur ungenau: meist wird das Normvolumen mit einer mittleren Temperatur und einem mittleren Druck berechnet und auch der Brennwert ist ein über die Abrechnungsperiode gemittelter Wert.

Zweck der Erfindung ist es, ein Verfahren und eine Messvorrichtung anzugeben, mit welchen spezifische Grössen für die Gasbeschaffenheit und/oder der Energieverbrauch zeitaktuell ermittelt werden können.

In der Veröffentlichung EP 0 715 169 A1 wird ein Verfahren und eine Messvorrichtung beschrieben, in welchen Messgrössen, wie Dichte, Viskosität, Schallgeschwindigkeit, Wärmeleitfähigkeit oder spezifische Wärme, eines Gases bestimmt werden, um daraus mittels Korrelationstabellen den Brennwert oder Wobbe-Index abzuleiten.

Aus EP 2 015 056 A1 ist ein Verfahren bekannt, in welchem das mit einem mikrothermischen Sensor bestimmte Verhältnis aus Wärmekapazität und Wärmeleitfähigkeit benutzt wird, um mittels Korrelation den Brennwert oder Wobbe-Index zu bestimmen.

Weiter ist aus WO 2004/008136 A1 ein Verfahren bekannt, in welchem die Wärmeleitfähigkeit, die Wärmekapazität und der Kohlendioxidanteil gemessen werden, um daraus mittels Korrelation den Brennwert zu bestimmen.

In WO 02/40992 A1 wird ein Verfahren beschrieben, in welchem aus der Wärmeleitfähigkeit bei zwei unterschiedlichen Temperaturen und der Schallgeschwindigkeit die Zusammensetzung eines Brenngases abgeleitet wird.

Aus der Patentanmeldung EP 14001767 ist ein Verfahren bekannt, bei dem ein Durchfluss mit einer kritischen Düse generiert wird, um mit einem nachgeschalteten mikrothermischen Sensor spezifische Grössen für die Gasbeschaffenheit zu bestimmen. Dieses Verfahren ist darauf angewiesen, dass über der Düse immer kritische Druckverhältnisse herrschen, sei es durch Beaufschlagung der Düse mit einem Vordruck oder durch Generieren eines Vakuums hinter der Düse. Das Verfahren ist deshalb nicht direkt zur Bestimmung von spezifischen Grössen für die Gasbeschaffenheit beim Endkunden geeignet, da die Versorgungsnetze an dieser Stelle selten den benötigten Vordruck aufweisen, und die Installation einer Vakuumpumpe hinter der Düse nicht in Frage kommt.

Aus EP 2 574 918 A1 ist ein Verfahren bekannt, bei dem ein mikrothermischer Sensor dazu verwendet wird, ein Volumendurchflussmessgerät dahingehend aufzuwerten, dass die Wärmediffusivität bestimmt werden kann, was bei bekannter Wärmeleitfähigkeit eine Einteilung der Gase in L(ow calorific)- beziehungsweise H(igh calorific)-Gase erlaubt. Aus dem Volumendurchfluss, der Wärmediffusivität und der Wärmeleitfähigkeit kann jedoch im Allgemeinen nicht mit genügender Genauigkeit auf den Brennwert und den Energiefluss geschlossen werden.

Aufgabe der vorliegenden Erfindung ist es, die Nachteile der erwähnten Verfahren zu beseitigen und ein Verfahren und eine Messvorrichtung vorzuschlagen, welche für Niederdruckgasnetze geeignet sind, und mittels welchen neben der Einteilung der Gase in L- bzw. H-Gase auch Brennwert und Energieverbrauch bestimmt werden können.

Diese Aufgabe wird durch ein Verfahren nach Anspruch 1 und durch eine Messvorrichtung nach Anspruch 7 gelöst.

Der Erfindung liegt die Idee zu Grunde, zur Bestimmung von spezifischen Grössen für die Gasbeschaffenheit und/oder des Energieverbrauchs einen Ultraschalldurchflusssensor mit einem mikrothermischen Sensor wie folgt zu kombinieren.

Messung der Schallgeschwindigkeit und des Volumendurchflusses mittels Ultraschalldurchflusssensors:

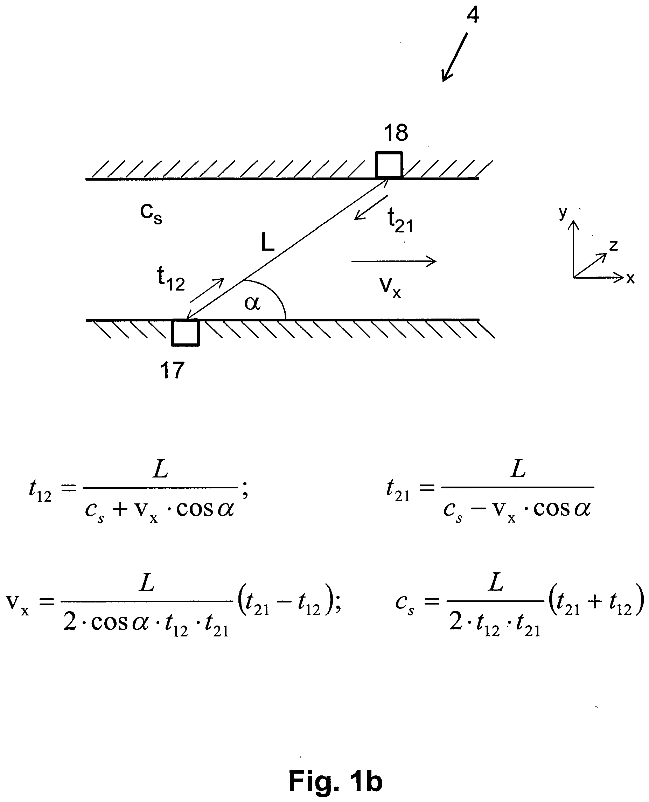

Zur Bestimmung des Volumendurchflusses wird typischerweise ein Ultraschallsignal in einem Winkel quer zur Strömungsrichtung ins Fliessmedium eingekoppelt und die Laufzeitdifferenz des Ultraschallsignals sowohl in als auch gegen die Strömungsrichtung gemessen (Fig. 1b). Die Laufzeitdifferenz der beiden Ultraschallsignale ist dann ein Mass für die mittlere Fliessgeschwindigkeit, aus der bei bekanntem Leitungsquerschnitt der Volumendurchfluss berechnet

werden kann:

$$\mathrm{v_x} = \frac{L}{2 \cdot \cos\alpha \cdot t_{12} \cdot t_{21}}(t_{21} - t_{12}); \qquad c_s = \frac{L}{2 \cdot t_{12} \cdot t_{21}}(t_{21} + t_{12}). \qquad (1)$$

wobei

$v_x$      die mittlere Fliessgeschwindigkeit,
$c_s$      die Schallgeschwindigkeit,
$L$      die Länge der Messstrecke,
$t_{12}$      die Laufzeit in Strömungsrichtung, und
$t_{21}$      die Laufzeit gegen die Strömungsrichtung bedeuten.

Die Summe der Laufzeiten enthält die Information zur Schallgeschwindigkeit $c_s$ des Mediums, die aber in Ultraschallzählern meist keine weitere Verwendung findet.

In Kombination mit einem mikrothermischen Sensor, wie in der Patentanmeldung EP 14001767 beschrieben, kann dank der Information zur Schallgeschwindigkeit auf die kritische Düse verzichtet werden, da letztere in erster Ordnung ebenfalls die Schallgeschwindigkeit liefert, mit dem Vorteil, dass keine kritischen Druckverhältnisse notwendig sind, d.h., dass bei gegebenem Druck gemessen werden kann. Somit werden in Niederdruckgasnetzen weder Verdichter noch Vakuumpumpen benötigt.

Bestimmung des Massenflusses:

Aus der Schallgeschwindigkeit kann durch Korrelation die Dichte bestimmt werden, die für die meisten Gase gut mit der Schallgeschwindigkeit korreliert. Um die Korrelation der Dichte weiter zu verbessern, kann zusätzlich die Wärmeleitfähigkeit bei einer oder mehreren Temperaturen gemessen und in die Korrelation einbezogen werden.

Der Massenfluss ist proportional zum Produkt $\rho \cdot v_x$ aus der Dichte $\rho$ und der Fliessgeschwindigkeit $v_x$:

$$\dot{m} = \rho \cdot \mathrm{v}_x \cdot A \ , \qquad (2)$$

wobei A der Querschnitt des Flusskanals bedeutet.

Messung der Wärmeleitfähigkeit mittels mikrothermischen Sensors:

Integrierte CMOS-Hitzdrahtanemometer ermöglichen sowohl eine mikrothermische Wärmeleitfähigkeitsmessung als auch eine Massenflussmessung. Zu dieser Technologie wird auf D. Matter, B. Kramer, T. Kleiner, B. Sabbattini, T. Suter, "Mikroelektronischer Haushaltsgaszähler mit neuer Technologie", Technisches Messen 71, 3 (2004), S. 137-146 hingewiesen.

Zur Beschreibung der mikrothermischen Messungen wird von der das mikrothermische System beschreibenden, eindimensionalen Wärmeleitungsgleichung ausgegangen (Kerson Huang: Statistical Mechanics, 2. Auflage, John Wiley & Sons, New York 1987, ISBN 0-471-85913-3):

$$\frac{c_p}{\lambda} \cdot \rho\, \mathrm{v}_x \cdot \frac{d}{dx}T = \nabla^2 T + \frac{1}{\lambda}\Theta \ , \qquad (3)$$

wobei

$v_x$      die Komponente der mittleren Fliessgeschwindigkeit $\vec{v}$ (Geschwindigkeitsvektor) in x-Richtung, d. h. entlang des Gasstromes,
$T$      die Temperatur,

$\dfrac{d}{dx}T$      der Temperaturgradient,

$c_p$      die Wärmekapazität des Gases bei konstantem Druck,

$\rho$      die Dichte,

$\lambda$      die Wärmeleitfähigkeit des Gases und

$\nabla^2 T$      der Laplace-Operator, angewandt auf die Temperatur *T,* wobei

$$\nabla^2 = \left(\frac{d}{d_x}\right)^2 + \left(\frac{d}{dy}\right)^2 + \left(\frac{d}{dz}\right)^2 ,$$

bedeuten. Da das Gas (Gasstrom) nur in x-Richtung fliesst, werden die Komponenten $v_y$ bzw. $v_z$ in y-Richtung bzw. in z-Richtung der mittleren Fliessgeschwindigkeit $\vec{v}$ zu Null angenommen. $\Theta$ mit der Einheit Watt/m³ beschreibt den Quellterm des Heizelements. Der Quellterm rührt bei der mikrothermischen Methode vom Heizdraht eines miniaturisierten, integrierten Hitzdrahtanemometers her, der Wärmeenergie in das System speist.

Man beachte, dass die Wärmeleitfähigkeit $\lambda$ wegen des Quellterms $\Theta$ separat auf die Lösung der Gleichung (3) einwirkt. Umgekehrt kann die Wärmeleitfähigkeit bestimmt werden, wenn der mikrothermische Sensor ohne beaufschlagten Massenfluss ($v_x = 0$ bzw. $\dot{m} = 0$) eingesetzt wird. Die zugehörige Differentialgleichung für die Temperaturverteilung lautet dann einfach

$$\nabla^2 T = -\frac{1}{\lambda}\Theta \; . \qquad\qquad (4)$$

Des Weiteren kann durch Variieren des Quellterms die Temperaturverteilung verändert werden, was die Bestimmung der Wärmeleitfähigkeit bei unterschiedlichen Temperaturen ermöglicht.

Bestimmung der Wärmekapazität mittels mikrothermischen Sensors:

Die Lösung der Gleichung (3), die die Temperaturverteilung im mikrothermischen System beschreibt, erlaubt es, durch das Messen dieser Temperaturverteilung den Flussfaktor $\varphi$,

$$\varphi := \frac{c_p}{\lambda} \cdot \rho \cdot v_x \; = \; \frac{c_p}{\lambda} \cdot \frac{\dot{m}}{A} \; , \qquad\qquad (5)$$

zu bestimmen, wobei A den Querschnitt des Flusskanals über dem mikrothermischen Sensor und $\dot{m}$ den Massenfluss bezeichnet. Mit bekanntem Massenfluss und bekannter Wärmeleitfähigkeit lässt sich schlussendlich die Wärmekapazität bestimmen.

Korrelation von spezifischen Grössen für die Gasbeschaffenheit:

Mit der Schallgeschwindigkeit $c_s$, der Wärmeleitfähigkeit $\lambda$ und der Wärmekapazität $c_p$ stehen drei unabhängige Messgrössen zur Verfügung, mit welchen nun spezifische Grössen Q für die Gasbeschaffenheit, wie z.B. der Brennwert, mittels einer Korrelationsfunktion $f_{corr}$ korreliert werden können:

$$Q \; = \; f_{corr}(sensor\ output) := f_{corr}(S_{out}) . \qquad (6)$$

Der "sensor Output" $S_{out}$ ist dabei eine Funktion der Ausgangsgrössen $c_s$, $\lambda$ und $c_p$:

$$S_{out} \; = \; f(c_s, \lambda, c_p) . \qquad\qquad (7)$$

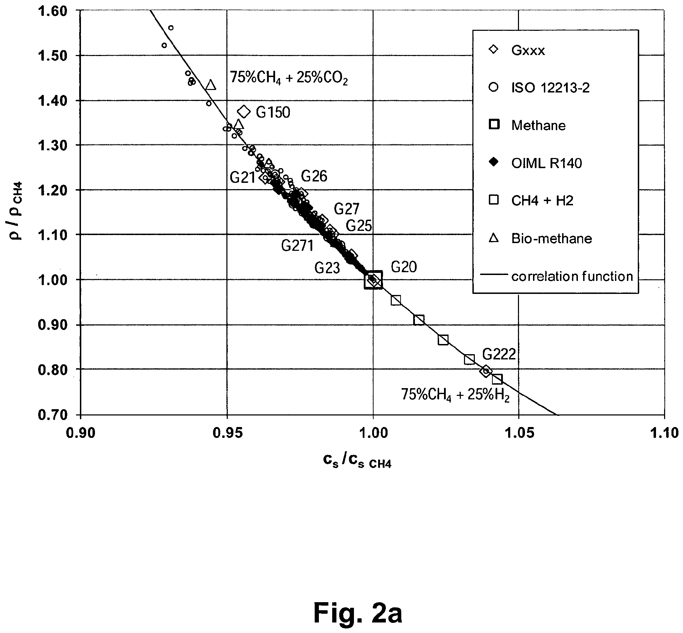

Beispielsweise ergibt sich für die Korrelation des in Fig. 2a gezeigten Dichteverhältnisses $Q = \rho / \rho_{ref}$ bei 0°C und 1013.25 mbar folgende Korrelationsfunktion

$$\rho_{corr}\Big/\rho_{ref} = f_{corr}(S_{out}) = a_0 + a_1 \cdot S_{out} + a_2 \cdot S_{out}^2 \qquad (8a)$$

mit Koeffizienten $a_0 = 36$, $a_1 = -65$ und $a_2 = 30$ und Methan (G20) als Referenz. $S_{out}$ ist dabei einfach die Schallgeschwindigkeit $c_s$:

$$S_{out} = f(c_s, \lambda, c_p) = c_s . \qquad (9a)$$

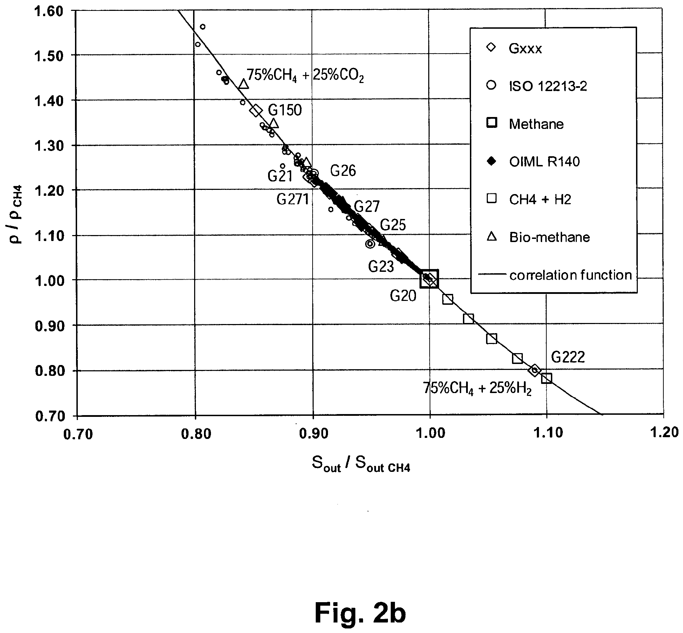

Fig. 2b zeigt eine verbesserte Korrelation des Dichteverhältnisses $Q = \rho / \rho_{ref}$ bei 0°C und 1013.25 mbar anhand der Schallgeschwindigkeit und der Wärmeleitfähigkeit, gemessen bei zwei verschiedenen Temperaturen.

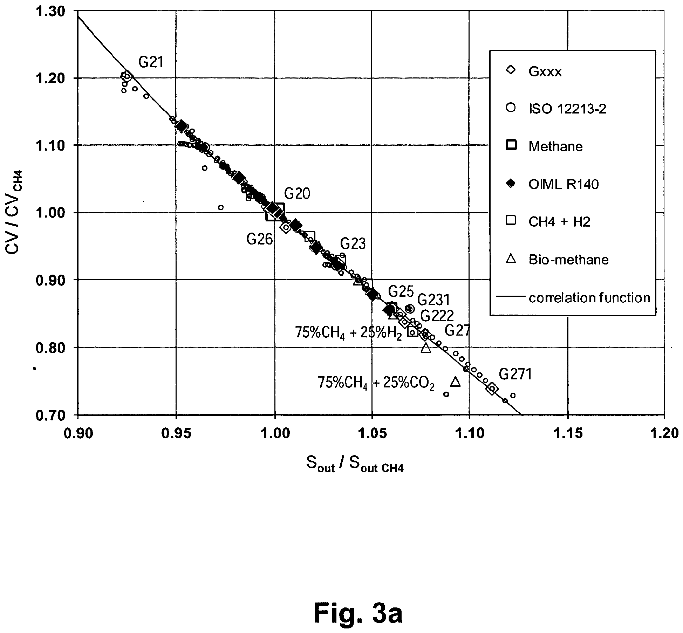

Im Falle der Korrelation von spezifischen Grössen für die Gasbeschaffenheit in Fig. 3a liest sich die Gleichung (8a) für das Beispiel des Brennwerts wie folgt:

$$CV\Big/CV_{ref} = f_{corr}(S_{out}) = a_0 + a_1 \cdot S_{out} + a_2 \cdot S_{out}^2 \qquad (8b)$$

mit Koeffizienten $a_0 = 8.1$, $a_1 = -11$ und $a_2 = 4.7$ und wiederum Methan (G20) als Referenz. $S_{out}$ ist nun eine Funktion aller drei Ausgangsgrössen:

$$S_{out} = f(c_s, \lambda, c_p) = \left(\frac{c_p}{c_{p,ref}}\right)^{-0.86} \cdot \left(\frac{\lambda}{\lambda_{ref}}\right)^{0.22} \cdot \frac{c_s}{c_{s,ref}} . \qquad (9b)$$

Aus den Ergebnissen in Fig. 2 und Fig. 3a ist leicht nachvollziehbar, dass mittels $c_s$, $c_p$ und $\lambda$ als weitere Gasbeschaffenheit der Wobbe-Index W als Mass für die Leistung eines Brenners, definiert als

$$W := \frac{CV}{\sqrt{\rho_{gas}\Big/\rho_{air}}} , \qquad (10)$$

korreliert werden kann, indem man die Gleichungen (8a) für die Dichte und (9b) für den Brennwert miteinander kombiniert.

Weiter kann als Gasbeschaffenheit aus den drei unabhängigen Grössen Schallgeschwindigkeit $c_s$, Wärmeleitfähigkeit $\lambda$ und Wärmekapazität $c_p$ zum Beispiel der Z- oder Realgas-Faktor korreliert werden, der die Abweichung des Verhaltens eines realen Gases vom idealen Gasgesetz beschreibt,

$$p \cdot V = Z \cdot R_m \cdot T . \qquad (11)$$

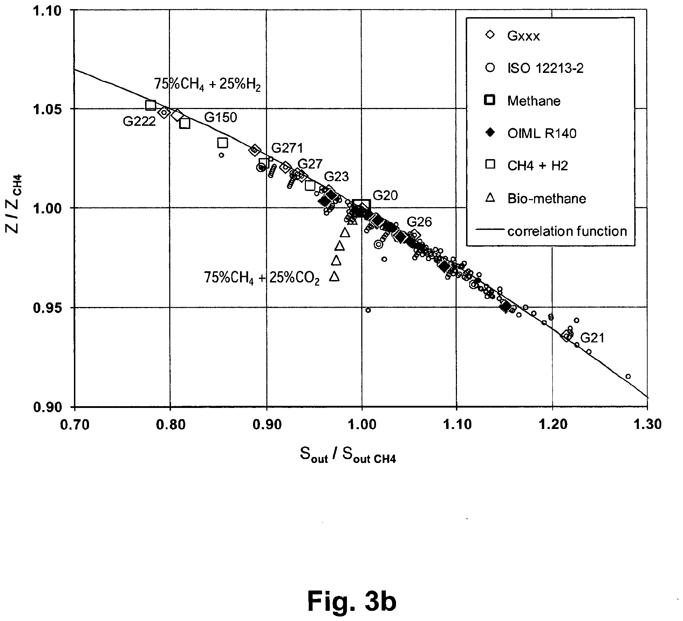

Das Realgasverhalten weicht speziell bei höheren Drücken stark vom idealen Gasverhalten ab, genau bei Drücken also, wie sie in den grossen Gastransportleitungen auftreten (und deshalb speziell beachtet werden müssen). Interessant bei dieser Anwendung ist die Tatsache, dass die Bestimmung der unabhängigen Grössen nicht etwa beim selben, hohen Druck erfolgen muss, sondern z.B. auch bei Umgebungsdruck stattfinden kann, wo der Aufbau einer entsprechenden Messvorrichtung viel einfacher zu bewerkstelligen ist. Fig. 3b zeigt eine mögliche Korrelation des Z-Faktors für 50 bar mit folgenden Eckdaten:

$$Z\Big/Z_{ref} = f_{corr}(S_{out}) = a_0 + a_1 \cdot S_{out} + a_2 \cdot S_{out}^2 + a_3 \cdot S_{out}^3 \qquad (8c)$$

mit Koeffizienten $a_0 = 1.1$, $a_1 = 0.15$, $a_2 = -0.29$ und $a_3 = 0.05$ und Methan (G20) als Referenz. $S_{out}$ ist wiederum eine Funktion aller drei Ausgangsgrössen (bei Umgebungsdruck):

$$S_{out} = f(c_s, \lambda, c_p) = (\frac{c_p}{c_{p,ref}})^{1.35} \cdot (\frac{\lambda}{\lambda_{ref}})^{-0.25} \cdot (\frac{c_s}{c_{s,ref}})^{-2} . \quad (9c)$$

Als weiteres Beispiel sei die Korrelation der kinematischen Viskosität, $\eta/\rho$ (Viskosität / Dichte) aufgeführt. Diese Grösse findet sich in der Reynoldszahl, Re, wieder, die in der Strömungslehre verwendet wird und als das Verhältnis von Trägheits- zu Zähigkeitskräften verstanden werden kann:

$$Re = \frac{\rho \cdot v \cdot d}{\eta} , \quad (12)$$

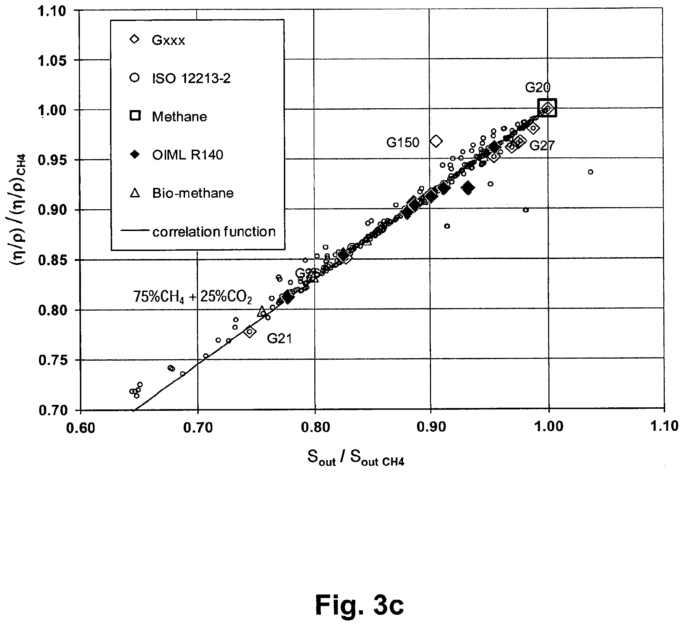

wobei $\rho$ die Dichte, v die Strömungsgeschwindigkeit des Gases gegenüber dem umströmten Körper und d die charakteristische Länge des Körpers bedeuten. Daraus ergibt sich, dass das Turbulenzverhalten geometrisch ähnlicher Körper bei gleicher Reynolds-Zahl identisch ist. Bei bekannter kinematischer Viskosität kann somit z.B. abgeschätzt werden, wann bei einem Gas in einem Rohleitungssystem Turbulenz auftritt, was bei Gasverteilnetzen ein wichtiger Input zur Auslegung solcher Netze ist. Fig. 3c zeigt die Korrelation zwischen der kinematischen Viskosität $\eta/\rho$ und dem Sensoroutput $S_{out}$:

$$\frac{(\eta/\rho)}{(\eta/\rho)_{ref}} = f_{corr}(S_{out}) = a_0 + a_1 \cdot S_{out} \quad (8d)$$

mit Koeffizienten $a_0$ =0.15 und $a_1$ = 0.85 und Methan (G20) als Referenz. $S_{out}$ ist wiederum eine Funktion aller drei Ausgangsgrössen:

$$S_{out} = f(c_s, \lambda, c_p) = (\frac{c_p}{c_{p,ref}})^{-0.65} \cdot (\frac{\lambda}{\lambda_{ref}})^{1.5} \cdot (\frac{c_s}{c_{s,ref}})^{1.5} . \quad (9d)$$

Es sei betont, dass die Wahl von $S_{out}$ einerseits, aber auch von $f_{corr}$ andererseits, keineswegs vorgegeben sind, sondern frei so gewählt werden, dass der resultierende Korrelationsfehler möglichst klein wird. Die in den Gleichungen (8a) bis (8d) aufgeführte Polynomfunktion ist eine typische Wahl, die meist zum Erfolg führt, während die Gleichungen (9a) bis (9d) eher den physikalischen Zusammenhang zu beschreiben versuchen.
Um zu zeigen, dass sich das Verfahren gemäss vorliegender Erfindung nicht auf die oben genannten Beispiele beschränkt, seien im Folgenden weitere Beispiele von spezifischen Grössen für die Gasbeschaffenheiten aufgeführt, die mit dem Verfahren bestimmt werden können:

Die Methanzahl, welche bei Gasmotorantrieben, seien diese stationär (z.B. in Wärme-Kraft-Koppelungsanlagen) oder im Mobilitätsbereich (z.B. in Gasfahrzeugen, Schiffen, etc.) eingesetzt, ein wichtiger Indikator für die Klopfneigung eines gasförmigen Treibstoffs ist.

Das "air to fuel ratio" und damit die dem Prozess zuzuführende Menge Luft. Die Kenntnis des "air to fuel ratio" ist zum Beispiel wichtig bei Verbrennungsvorgängen, seien diese stöchiometrisch (z.B. in Feuerungsanlagen) oder mit Luftüberschuss (z.B. in Magermotoren), mit offener Flamme oder katalytischer Art (z.B. in Reformationsprozessen in Hochtemperatur-Brennstoffzellen), um sowohl die Effizienz des Verbrennungsprozesses als auch das Abgasverhalten zu optimieren.

Der Methangehalt, dessen Überwachung z.B. in der Prozessindustrie von Bedeutung ist. In Biogasanlagen wird der Methangehalt typisch im Rohbiogas (z.B. als Mass für die Fermentereffizienz) und/oder im Gas, das in das Erdgasnetz eingespeist werden soll, (z. B. zur Qualitätskontrolle) und/oder im in die Luft abgelassenen Restgas (vorwiegend Kohlendioxid mit möglichst wenig Methan, da letzteres einen hohen Treibhauseffekt aufweist) überwacht.

Verfahrensschritte in einem typischen Ausführungsbeispiel

1. Messen von Druck p und Temperatur T des Gases.

2. Ultraschallbestimmung des zur Fliessgeschwindigkeit $v_x$ proportionalen Volumendurchflusses und der Schallgeschwindigkeit $c_s$, die für die meisten Gase gut mit der Normdichte $\rho_{norm}$ korreliert.

3. Einbezug der mit dem mikrothermischen Sensor gemessenen Wärmeleitfähigkeit $\lambda_{Ti}$ (bei einer oder mehreren Temperaturen $T_i$), um die Korrelation der Normdichte $\rho_{norm}$ weiter zu verbessern.

4. Berechnung der Dichte bei Betriebsbedingungen gemäss

$$\rho = \rho_{norm} \cdot \frac{T_{norm} \cdot p}{p_{norm} \cdot T} \, . \qquad (13)$$

5. Benutzen dieser Information ($v_x$, $\rho$) zur Bestimmung des Massenflusses $\dot{m}$ proportional zu $\rho \cdot v_x$ und, zusammen mit der Wärmeleitfähigkeit $\lambda$ und dem mit dem mikrothermischen Sensor gemessenen Flussfaktor $\varphi$, der Wärmekapazität $c_p$.

6. Korrelieren der gewünschten spezifische Grösse für die Gasbeschaffenheit, insbesondere des Brennwerts CV, aus Schallgeschwindigkeit $c_s$, Wärmeleitfähigkeit $\lambda$ und Wärmekapazität $c_p$.

7. Bei Bedarf kann aus der Multiplikation von Massen- bzw. Volumenfluss mit dem Brennwert CV (in J/kg bzw. J/m$^3$) der Energieverbrauch $\Phi_{En}$ bestimmt werden.

Unter der oben erwähnten Normdichte $\rho_{norm}$ wird in dieser Beschreibung die Dichte bei einer festgelegten Temperatur $T_{norm}$ und einem festgelegten Druck $p_{norm}$ verstanden. Die Normdichte wird üblicherweise bei 0°C und 1013.25 mbar angegeben. Es ist jedoch auch möglich, andere Werte für die Temperatur $T_{norm}$ und den Druck $p_{norm}$ festzulegen, für welche die Korrelation zwischen Dichte und Schallgeschwindigkeit bekannt ist.

Verfahren und Messvorrichtung gemäss vorliegender Erfindung

In dem Verfahren zur Bestimmung von spezifischen Grössen für die Gasbeschaffenheit gemäss vorliegender Erfindung

- fliesst ein Gas oder Gasgemisch durch einen Ultraschalldurchflusssensor und über einen mikrothermischen Sensor, wobei

- Temperatur und Druck des Gases oder Gasgemisches erfasst werden;

- mit dem Ultraschalldurchflusssensor sowohl die Fliessgeschwindigkeit bzw. der Volumenfluss als auch die Schallgeschwindigkeit des Gases oder Gasgemisches bestimmt werden;

- aus der Schallgeschwindigkeit die Dichte des Gases oder Gasgemisches korreliert wird;

- die Dichteinformation, zusammen mit der Fliessgeschwindigkeit, zur Berechnung des Massenflusses verwendet werden;

- mit Hilfe des mikrothermischen Sensors die Wärmeleitfähigkeit des Gases oder Gasgemisches bei einer oder mehreren Temperaturen bestimmt wird;

- aus dem Durchflusssignal des mikrothermischen Sensors der Flussfaktor berechnet wird, um aus demselben, zu-

7

sammen mit der Information des Massenflusses und der Wärmeleitfähigkeit, die Wärmekapazität oder eine von der Wärmekapazität abhängige Grösse des Gases oder Gasgemisches zu bestimmen;

- schliesslich die Schallgeschwindigkeit, die Wärmeleitfähigkeit bei einer oder mehreren Temperaturen und entweder die Wärmekapazität oder die von der Wärmekapazität abhängige Grösse zur Korrelation von spezifischen Grössen für die Gasbeschaffenheit, insbesondere des Brennwerts, verwendet werden.

Die mit dem Ultraschalldurchflusssensor bestimmte Schallgeschwindigkeit kann bei Bedarf auf die Schallgeschwindigkeit bei Normtemperatur umgerechnet werden.

In einer vorteilhaften Ausführungsvariante wird die mit Hilfe des mikrothermischen Sensors bestimmte Wärmeleitfähigkeit bei einer oder mehreren Temperaturen, zusammen mit der Schallgeschwindigkeit, zur genaueren Korrelation der Dichte verwendet.

Die aus der Schallgeschwindigkeit bzw. aus der Schallgeschwindigkeit und Wärmeleitfähigkeit korrelierte Dichte kann beispielsweise die Normdichte sein. Vorteilhafterweise wird die aus der Schallgeschwindigkeit bzw. aus der Schallgeschwindigkeit und Wärmeleitfähigkeit korrelierte Dichte oder Normdichte mit der Temperatur und dem Druck des Gases oder Gasgemisches auf die Dichte bei Betriebsbedingungen umgerechnet.

In einer vorteilhaften Ausführungsform des Verfahrens werden die Schallgeschwindigkeit, die Wärmeleitfähigkeit bei einer oder mehreren Temperaturen und entweder die Wärmekapazität oder die von der Wärmekapazität abhängige Grösse zur Korrelation des Brennwerts oder Wobbe-Index (W) oder Z-Faktors oder der kinematischen Viskosität verwendet.

In einer weiteren vorteilhaften Ausführungsform des Verfahrens wird aus dem Brennwert, zusammen mit dem Volumen- oder Massenfluss, der Energieverbrauch errechnet, beispielsweise indem das Produkt aus Volumen- bzw. Massenfluss und Brennwert zeitlich aufintegriert wird.

Das oben beschriebene Verfahren und die oben beschriebenen Ausführungsformen und -varianten eignen sich sowohl für die kontinuierliche als auch für die intermittierende Bestimmung von spezifischen Grössen für die Gasbeschaffenheit und/oder des Energieverbrauchs.

Die Messvorrichtung zur Bestimmung von spezifischen Grössen für die Gasbeschaffenheit und/oder des Energieverbrauchs gemäss vorliegender Erfindung umfasst eine Auswerteeinheit, die zur Ausführung eines Verfahrens gemäss einer der oben beschriebenen Ausführungsformen und -varianten eingerichtet ist, sowie einen Ultraschalldurchflusssensor zur Messung von Schall- und Fliessgeschwindigkeit, einen Drucksensor zur Messung des Drucks, einen Temperatursensor zur Messung der Temperatur und einen mikrothermischen Sensor zur Messung der Wärmeleitfähigkeit und entweder der Wärmekapazität oder einer von der Wärmekapazität abhängigen Grösse des Gases oder Gasgemisches.

In einer ersten Ausführungsform der Messvorrichtung sind der Ultraschalldurchflusssensor und der mikrothermische Sensor in einer Gasleitung angeordnet und können mit demselben Massenstrom beaufschlagt werden.

In einer zweiten Ausführungsform der Messvorrichtung sind der Ultraschalldurchflusssensor in einer Hauptgasleitung und der mikrothermische Sensor in einer Bypassgasleitung zur Hauptgasleitung angeordnet, wobei in der Hauptgasleitung ein Druckabfall erzeugendes Element vorgesehen ist, um in der Bypassgasleitung einen Massenstrom zu erwirken.

Mit Vorteil ist der Ultraschalldurchflusssensor in der ersten und zweiten Ausführungsform nicht invasiv auf die Gasleitung oder Hauptgasleitung aufgesetzt.

In einer dritten Ausführungsform der Messvorrichtung sind der Ultraschalldurchflusssensor und der mikrothermische Sensor in einer Bypassgasleitung zu einer Hauptgasleitung angeordnet, wobei in der Hauptgasleitung ein Druckabfall erzeugendes Element vorgesehen ist, um in der Bypassgasleitung einen Massenstrom zu erwirken.

Mit Vorteil ist in der zweiten und dritten Ausführungsform das Teilungsverhältnis zwischen Massenfluss in der Bypass- und in der Hauptgasleitung bekannt, beispielsweise durch Kalibrieren mit einem bekannten Gas.

Unabhängig von der Ausführungsform und -variante kann die Messvorrichtung zusätzlich einen Abschnitt der Gasleitung oder Hauptgasleitung und/oder eine Bypassgasleitung, in denen mindestens einer der Sensoren der Messvorrichtung angeordnet ist, und/oder ein Druckabfall erzeugendes Element in der Hauptgasleitung umfassen.

Vorteilhafterweise bildet die Auswerteeinheit zusammen mit der restlichen Messvorrichtung eine Baueinheit. Je nach Anwendung kann die Messvorrichtung auch ohne die Auswerteeinheit eine Baueinheit bilden, wobei die Auswerteeinheit in einer separaten oder übergeordneten Recheneinheit ausgebildet sein kann.

Das Verfahren und die Messvorrichtung gemäss vorliegender Erfindung zur Bestimmung von spezifischen Grössen für die Gasbeschaffenheit und/oder des Energieverbrauchs haben den Vorteil, dass sie auch in Niederdruckgasnetzen verwendet werden können, ohne dass, wie in der in der Patentanmeldung EP 14001767 beschriebenen Messvorrichtung, ein zusätzlicher Verdichter oder eine zusätzliche Vakuumpumpe erforderlich ist.

Weiter vorteilhaft ist, dass die mit Hilfe des mikrothermischen Sensors bestimmte Wärmeleitfähigkeit des Gases oder Gasgemisches bei einer oder mehreren Temperaturen, zusammen mit der Schallgeschwindigkeit, zur genaueren Korrelation der Dichte verwendet werden kann, was zu genaueren Werten für den Massenfluss führt.

Die Korrelation von spezifischen Grössen für die Gasbeschaffenheit aus den drei unabhängigen Variablen Schallgeschwindigkeit, Wärmeleitfähigkeit und Wärmekapazität ermöglicht zudem eine höhere Genauigkeit bei der Bestimmung des Brennwerts und Energieverbrauchs, als dies mit dem eingangs beschriebenen Verfahren nach EP 2 574 918 A1 möglich ist.

Die Erfindung wird im Folgenden anhand der Zeichnungen näher erläutert. Es zeigen:

Fig. 1a    den schematischen Aufbau eines Ausführungsbeispiels eines mikrothermischen Anemometers,

Fig. 1b    eine schematische Darstellung eines Ultraschalldurchflusssensors,

Fig. 2a    ein Beispiel einer Dichtebestimmung (Korrelation) anhand der Schallgeschwindigkeit,

Fig. 2b    ein Beispiel einer verbesserten Dichtebestimmung (Korrelation) anhand der Schallgeschwindigkeit und der Wärmeleitfähigkeit,

Fig. 3a    ein Beispiel einer Brennwertbestimmung (Korrelation) anhand der Wärmekapazität, der Wärmeleitfähigkeit und der Schallgeschwindigkeit,

Fig. 3b    ein Beispiel für die Bestimmung des Z-Faktors (Korrelation) anhand der Wärmekapazität, der Wärmeleitfähigkeit und der Schallgeschwindigkeit,

Fig. 3c    ein Beispiel für die Bestimmung der kinematischen Viskosität (Korrelation) anhand der Wärmekapazität, der Wärmeleitfähigkeit und der Schallgeschwindigkeit,

Fig. 4     ein Ausführungsbeispiel des schematischen Aufbaus einer Messvorrichtung gemäss vorliegender Erfindung in der Hauptgasleitung,

Fig. 5     ein zweites Ausführungsbeispiel des schematischen Aufbaus einer Messvorrichtung gemäss vorliegender Erfindung mit einem mikrothermischen Sensor in einer Bypassgasleitung zur Hauptgasleitung und

Fig. 6     ein drittes Ausführungsbeispiel des schematischen Aufbaus einer Messvorrichtung gemäss vorliegender Erfindung in einer Bypassgasleitung.

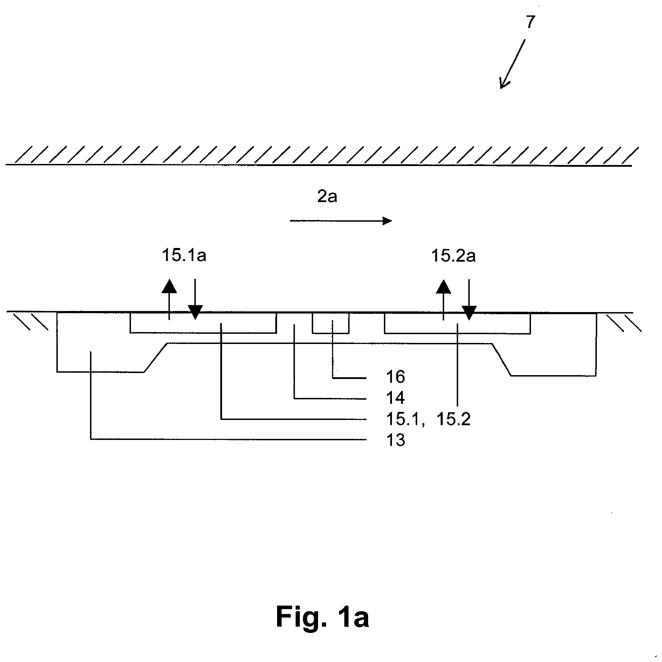

Fig. 1a zeigt ein Ausführungsbeispiel eines mikrothermischen Sensors 7 zur Verwendung in einer Messvorrichtung gemäss vorliegender Erfindung. Der mikrothermische Sensor kann beispielsweise, wie in Fig. 1a gezeigt, ein integriertes, mikrothermisches CMOS-Hitzdrahtanemometer sein, das im Einsatz in einem Abschnitt einer Bypassgasleitung angeordnet und mit einem Gas- oder Gasgemischstrom 2a beaufschlagt werden kann. Das mikrothermische CMOS-Hitzdrahtanemometer umfasst ein Substrat 13, das typisch eine Membran 14 von wenigen Mikrometer Dicke enthält. Weiter umfasst das CMOS-Hitzdrahtanemometer zwei Thermoelemente 15.1, 15.2 und ein Heizelement 16, das in Flussrichtung zwischen den beiden Thermoelementen angeordnet sein kann. Mit den beiden Thermoelementen 15.1, 15.2 kann die Temperatur erfasst werden, die sich auf Grund des Wärmeaustausches 15.1a, 15.2a mit dem Gas- oder Gasgemischstrom 2a einstellt.

Für weitere Einzelheiten zur Funktionsweise des integrierten, mikrothermischen CMOS-Hitzdrahtanemometers wird auf D. Matter, B. Kramer, T. Kleiner, B. Sabbattini, T. Suter, "Mikroelektronischer Haushaltsgaszähler mit neuer Technologie", Technisches Messen 71, 3 (2004), S. 137-146 verwiesen.

Fig. 1b zeigt ein Ausführungsbeispiel eines Ultraschalldurchflusssensors 4 zur Verwendung in einer Messvorrichtung gemäss vorliegender Erfindung. Beispielsweise sind zwei sowohl Schall erzeugende als auch Schall aufnehmende Einheiten 17 und 18 (z.B. Piezoaktoren bzw. -rezeptoren) an schräg gegenüberliegenden Positionen an der Messleitung angeordnet. Ein vom Aktor 17 ausgesendeter Schallimpuls erreicht den Rezeptor 18 schneller, als ein gleichzeitig vom Aktor 18 ausgesendeter Schallimpuls den Rezeptor 17 erreicht. Aus den Laufzeiten $t_{12}$ und $t_{21}$ lassen sich, zusammen mit Geometriefaktoren der Anordnung, sowohl die Schallgeschwindigkeit $c_s$ als auch die Fliessgeschwindigkeit $v_x$ errechnen.

Für weitere Einzelheiten zur Funktionsweise des Ultraschallsensors wird auf L.C. Lynnwortha, Yi Liub, "Ultrasonic flowmeters: Half-century progress report, 1955-2005" in Ultrasonics, 44, Supplement (2006), S. e1371-e1378, verwiesen.

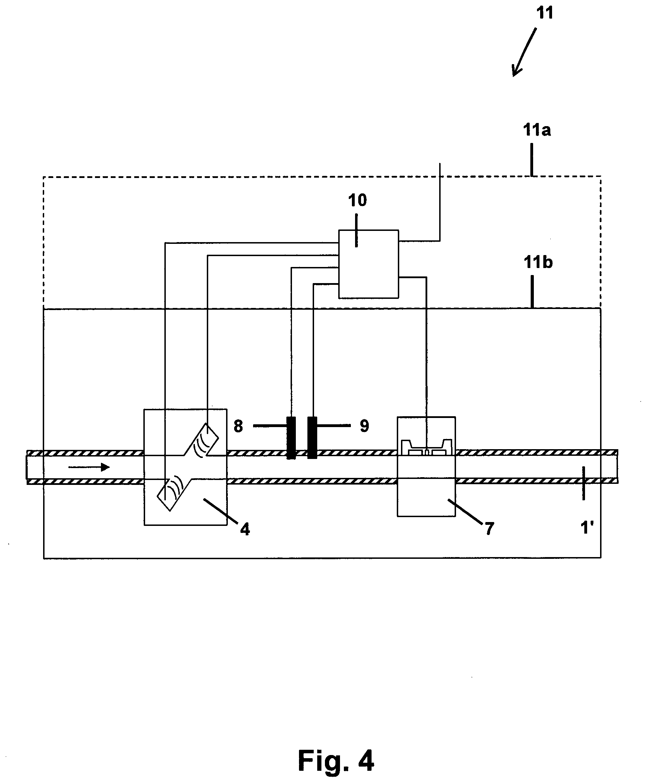

Fig. 4 zeigt ein Ausführungsbeispiel des schematischen Aufbaus einer Messvorrichtung gemäss vorliegender Erfindung. Im Ausführungsbeispiel umfasst die Messvorrichtung 11 eine Auswerteeinheit 10, die zur Ausführung eines Verfahrens gemäss vorliegender Erfindung eingerichtet ist, einen Ultraschalldurchflusssensor 4, einen mikrothermischen Sensor 7 sowie einen Drucksensor 8 und einen Temperatursensor 9, wobei die Sensoren in einer Gasleitung 1' angeordnet sein können. Einzelne dieser Komponenten oder alle diese Komponenten können zu einer Baueinheit zusammengefasst werden, wobei die Auswerteeinheit 10 Bestandteil dieser Baueinheit sein kann (Variante 11a), oder die Auswerteeinheit kann separat dazu beigestellt werden (Variante 11b), z.B. in einer übergeordneten Recheneinheit.

Der Aufbau in dem in Fig. 4 gezeigten Ausführungsbeispiel eignet sich v.a. für die Bestimmung von spezifischen Grössen für die Gasbeschaffenheit bei kleinen und kleinsten Gasdurchflüssen, wie sie z.B. im Bereich der Gasanalytik auftreten, und wo primär die Information bezüglich der Gasbeschaffenheit wichtig ist.

Die Messvorrichtung in dem in Fig. 4 gezeigten Ausführungsbeispiel kann beispielsweise als Analyseeinheit oder als eigenständiges Analysegerät eingesetzt werden, wobei die Analyseeinheit oder das Analysegerät vorteilhafterweise eine Gasleitung 1' enthält, in welcher die Sensoren 4, 7, 8, 9 der Messvorrichtung angeordnet sind. Mit der Analyseeinheit bzw. dem Analysegerät können Gasproben entnommen und analysiert werden. Die hierfür notwendigen Anschlüsse und Ventile sind in Fig. 4 nicht dargestellt.

Ein Ausführungsbeispiel des Verfahrens zur Bestimmung von spezifischen Grössen für die Gasbeschaffenheit eines Gases und Gasgemisches gemäss vorliegender Erfindung wird im Folgenden anhand von Fig. 4 beschrieben. In dem Verfahren fliesst das Gas oder Gasgemisch in der Gasleitung 1' durch einen Ultraschalldurchflusssensor 4 und über einen mikrothermischen Sensor 7. Mit einem zusätzlich in der Gasleitung angeordneten Drucksensor 8 und Temperatursensor 9 werden Druck und Temperatur des Gases oder Gasgemisches, d.h. die Betriebsbedingungen, bestimmt. Weiter wird mit dem Ultraschallsensor die Schallgeschwindigkeit und die Fliessgeschwindigkeit bzw. der Volumenfluss gemessen. Anschliessend erfolgt die Korrelation der Dichte anhand der Schallgeschwindigkeit, wobei die mittels Korrelation ermittelte Dichte zweckmässigerweise auf die Dichte bei der gegebenen Temperatur und dem gegebenen Druck (Betriebsbedingungen) umgerechnet wird.

Zudem wird mit dem mikrothermischen Sensor 7 die Wärmeleitfähigkeit des Gases bei einer oder mehreren Temperaturen gemessen, indem die Heizleistung des Hitzdrahtes variiert wird. Wenn nötig, kann das Ergebnis dieser Messung auch in die Korrelation der Dichte einbezogen werden. Mit dem Wert der Dichte und dem Volumenstrom wird in Folge der Massenstrom berechnet. Aus dem ebenfalls mit dem mikrothermischen Sensor gemessenen Flussfaktor wird das Verhältnis zwischen Wärmekapazität und Wärmeleitfähigkeit des Gases und, zusammen mit der bereits bekannten Wärmeleitfähigkeit, der Wert der Wärmekapazität berechnet. Schallgeschwindigkeit, Wärmeleitfähigkeit und Wärmekapazität werden anschliessend zur Korrelation der spezifischen Grössen für die Gasbeschaffenheit, beispielsweise des Brennwerts oder Wobbe-Index (W) oder Z-Faktors oder der kinematischen Viskosität, verwendet. Durch Multiplikation des Massenstroms mit dem Brennwert kann bei Bedarf der Energieverbrauch bestimmt werden.

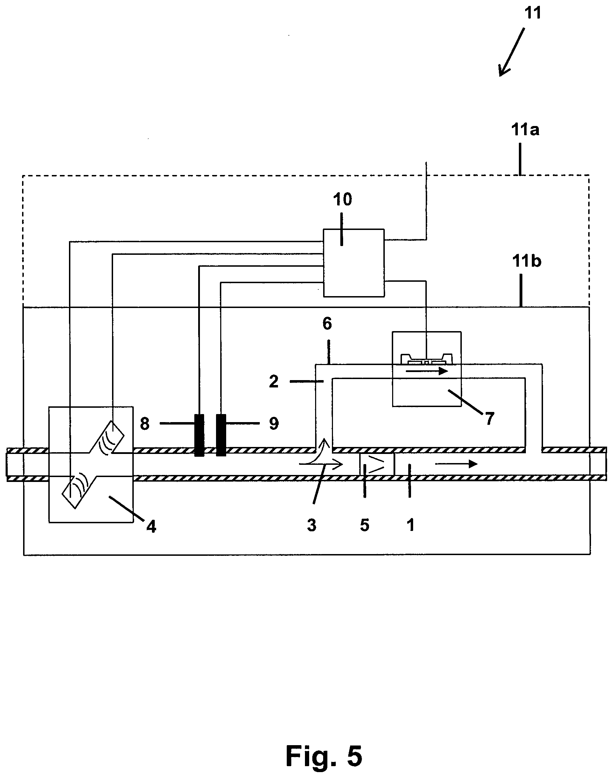

Fig. 5 zeigt ein zweites Ausführungsbeispiel des schematischen Aufbaus einer Messvorrichtung 11 gemäss vorliegender Erfindung mit einem mikrothermischen Sensor 7 in einer Bypassgasleitung 6 zur Hauptgasleitung 1. In der Hauptgasleitung ist in diesem Fall ein Druckabfall erzeugendes Element 5 vorgesehen, sodass sich im Betrieb ein Druckabfall über der Bypassgasleitung ausbildet, was zu einem Gasfluss 2 in der Bypassgasleitung führt, wobei sich ein charakteristisches Durchflussteilungsverhältnis 3 zwischen Haupt- und Bypassgasleitung einstellt.

Im gezeigten Ausführungsbeispiel umfasst die Messvorrichtung, zusätzlich zum mikrothermischen Sensor 7, eine Auswerteeinheit 10, die zur Ausführung eines Verfahrens gemäss vorliegender Erfindung eingerichtet ist, sowie einen Ultraschalldurchflusssensor 4, einen Drucksensor 8 und einen Temperatursensor 9, die typisch in der Hauptgasleitung 1 angeordnet sind. Einzelne dieser Komponenten oder alle diese Komponenten können zu einer Baueinheit zusammengefasst werden, wobei die Auswerteeinheit 10 Bestandteil dieser Baueinheit sein kann (Variante 11a), oder die Auswerteeinheit separat dazu beigestellt werden kann (Variante 11b), z.B. in einer übergeordneten Recheneinheit.

Der Aufbau in dem in Fig. 5 gezeigten Ausführungsbeispiel eignet sich sowohl für die Bestimmung von spezifischen Grössen für die Gasbeschaffenheit als auch, im Falle des Brennwerts als Gasbeschaffenheit, für die Energieverbrauchsmessung bei mittleren bis grossen Gasdurchflüssen, wie sie z.B. im Haushaltsbereich, in der Industrie oder im eichpflichtigen Verkehr auftreten.

Der Ultraschalldurchflusssensor 4 muss dabei nicht unbedingt in die Gasleitung 1' oder Hauptgasleitung 1 eingebaut sein, sondern kann auch als sogenanntes "Clamp-on Device" von aussen her auf die Gasleitung oder Hauptgasleitung aufgebracht sein. Der mikrothermische Sensor 7 hingegen benötigt nur kleinste Durchflussmengen und wird deshalb vorzugsweise in einer Bypassgasleitung 6 angeordnet.

Ein zweites Ausführungsbeispiel des Verfahrens zur Bestimmung von spezifischen Grössen für die Gasbeschaffenheit eines Gases und Gasgemisches gemäss vorliegender Erfindung wird im Folgenden anhand von Fig. 5 beschrieben. In dem Verfahren fliesst das Gas oder Gasgemisch in einer Hauptgasleitung 1 über oder durch ein Druckabfall erzeugendes Element 5. Vor dem Druckabfall erzeugenden- Element 5 zweigt eine Bypassgasleitung 6 ab, um sich nach demselben wieder mit der Hauptgasleitung zu vereinen. Durch das Druckabfall erzeugende Element 5 wird ein Teil des Gases oder Gasgemisches 2 gezwungen, durch die Bypassgasleitung 6 und über einen darin angeordneten mikrothermischen Sensor 7 zu fliessen. Der Ultraschalldurchflusssensor 4 wird mit dem Hauptgasfluss beaufschlagt.

Mit einem zusätzlich in der Hauptgasleitung angeordneten Drucksensor 8 und Temperatursensor 9 werden Druck und Temperatur des Gases oder Gasgemisches, d.h. die Betriebsbedingungen, bestimmt. Weiter wird mit dem Ultraschallsensor die Schallgeschwindigkeit und die Fliessgeschwindigkeit bzw. der Volumenfluss gemessen. Anschliessend erfolgt die Korrelation der Dichte anhand der Schallgeschwindigkeit, wobei die mittels Korrelation ermittelte Dichte zweckmässigerweise auf die Dichte bei der gegebenen Temperatur und beim gegebenen Druck (Betriebsbedingungen) umgerechnet wird.

Zudem wird mit dem mikrothermischen Sensor 7 die Wärmeleitfähigkeit des Gases bei einer oder mehreren Temperaturen gemessen, indem die Heizleistung des Hitzdrahtes variiert wird. Wenn nötig, kann das Ergebnis dieser Messung auch in die Korrelation der Dichte einbezogen werden. Mit dem Wert der Dichte und dem Volumenstrom wird in Folge der Massenstrom durch die Hauptgasleitung 1 berechnet. Anschliessend wird zweckmässigerweise auf das Teilungsverhältnis des Massenflusses zwischen Haupt- und Bypassgasleitung zurückgegriffen, um den Massenstrom in der Bypassgasleitung zu berechnen. Das Teilungsverhältnis kann beispielsweise im Voraus in einer Kalibriermessung mit bekannten Gasen bestimmt werden.

Aus dem ebenfalls mit dem mikrothermischen Sensor gemessenen Flussfaktor wird das Verhältnis zwischen Wärmekapazität und Wärmeleitfähigkeit des Gases oder Gasgemisches und, mit der bereits bekannten Wärmeleitfähigkeit, der Wert der Wärmekapazität berechnet. Schallgeschwindigkeit, Wärmeleitfähigkeit und Wärmekapazität werden anschliessend zur Korrelation der spezifischen Grössen für die Gasbeschaffenheit verwendet. Im Falle des Brennwerts als Gasbeschaffenheit liefert die Multiplikation des Massenstroms in der Hauptgasleitung mit dem Brennwert zusätzlich den Energieverbrauch.

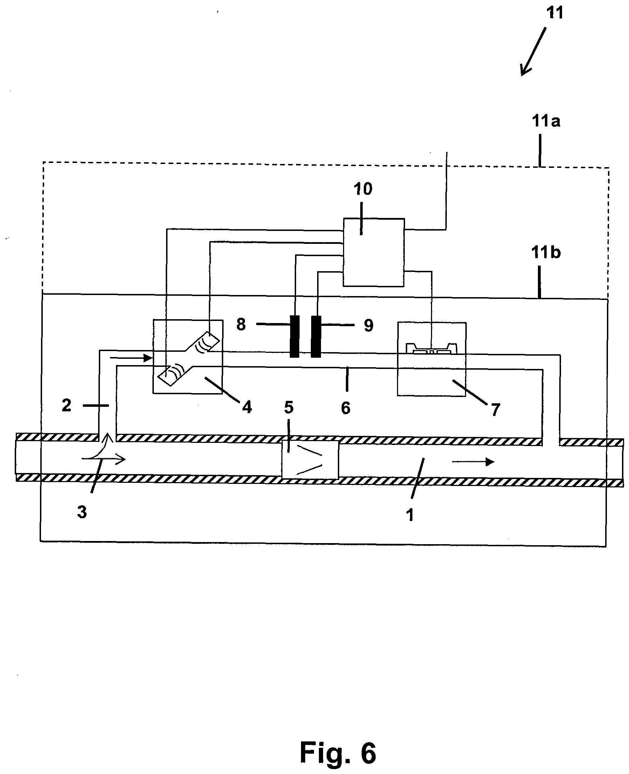

Fig. 6 zeigt ein drittes Ausführungsbeispiel des schematischen Aufbaus einer Messvorrichtung 11 gemäss vorliegender Erfindung in einer Bypassgasleitung 6 zur Hauptgasleitung 1. In der Hauptgasleitung ist in diesem Fall ein Druckabfall erzeugendes Element 5 vorgesehen, sodass sich im Betrieb ein Druckabfall über der Bypassgasleitung ausbildet, was zu einem Gasfluss 2 in der Bypassgasleitung führt, wobei sich ein charakteristisches Durchflussteilungsverhältnis 3 zwischen Haupt- und Bypassgasleitung einstellt.

Im gezeigten Ausführungsbeispiel umfasst die Messvorrichtung eine Auswerteeinheit 10, die zur Ausführung eines Verfahrens gemäss vorliegender Erfindung eingerichtet ist, sowie einen Ultraschalldurchflusssensor 4 und einen mikrothermischen Sensor 7, die in der Bypassgasleitung 6 angeordnet sind. Weiter umfasst die Messvorrichtung einen Drucksensor 8 und einen Temperatursensor 9, die meist ebenfalls in der Bypassgasleitung 1 angeordnet sind. Einzelne dieser Komponenten oder alle diese Komponenten können zu einer Baueinheit zusammengefasst werden, wobei die Auswerteeinheit 10 Bestandteil dieser Baueinheit sein kann (Variante 11a), oder die Auswerteeinheit separat dazu beigestellt werden kann (Variante 11b), z.B. in einer übergeordneten Recheneinheit.

Der Aufbau in dem in Fig. 6 gezeigten Ausführungsbeispiel ergibt sich vorzugsweise dann, wenn auch der Ultraschallsensor 4 in Mikrotechnik aufgebaut ist und dieser wie auch der mikrothermische Sensor 7 nur kleinste Durchflussmengen benötigt. Vorteilhafterweise werden dann beide Sensoren in einer Bypassgasleitung 6 angeordnet.

Ein drittes Ausführungsbeispiel des Verfahrens zur Bestimmung von spezifischen Grössen für die Gasbeschaffenheit eines Gases und Gasgemisches gemäss vorliegender Erfindung wird im Folgenden anhand von Fig. 6 beschrieben. Das Verfahren eignet sich sowohl für die kontinuierliche als auch für die intermittierende Bestimmung von spezifischen Grössen für die Gasbeschaffenheit und/oder des Energieverbrauchs. Gegebenenfalls notwendige Anschlüsse und Ventile sind in Fig. 6 nicht dargestellt.

Im dritten Ausführungsbeispiel des Verfahrens fliesst das Gas oder Gasgemisch in einer Hauptgasleitung 1 über oder durch ein Druckabfall erzeugendes Element 5. Vor dem Druckabfall erzeugenden Element 5 zweigt eine Bypassgasleitung 6 ab, um sich nach demselben wieder mit der Hauptgasleitung zu vereinen. Durch das Druckabfall erzeugende Element wird ein Teil des Gases oder Gasgemisches 2 gezwungen, durch die Bypassgasleitung 6 und durch einen Ultraschalldurchflusssensor 4 und über einen mikrothermischen Sensor 7 zu fliessen, die in der Bypassgasleitung angeordnet sind. Dabei werden der Ultraschalldurchflusssensor 4 und der mikrothermischen Sensor 7 mit demselben Gasfluss beaufschlagt.

Mit einem zusätzlich in der Bypassgasleitung angeordneten Drucksensor 8 und Temperatursensor 9 werden Druck und Temperatur des Gases oder Gasgemisches, d.h. die Betriebsbedingungen, bestimmt. Weiter wird mit dem Ultraschallsensor die Schallgeschwindigkeit und die Fliessgeschwindigkeit bzw. der Volumenfluss gemessen. Anschliessend erfolgt die Korrelation der Dichte anhand der Schallgeschwindigkeit, wobei die mittels Korrelation ermittelte Dichte zweckmässigerweise auf die Dichte bei der gegebenen Temperatur und dem gegebenen Druck (Betriebsbedingungen) umgerechnet wird.

Zudem wird mit dem mikrothermischen Sensor 7 die Wärmeleitfähigkeit des Gases bei einer oder mehreren Temperaturen gemessen, indem die Heizleistung des Hitzdrahtes variiert wird. Wenn nötig, kann das Ergebnis dieser Messung auch in die Korrelation der Dichte einbezogen werden. Mit dem Wert der Dichte und dem Volumenstrom wird in Folge der Massenstrom durch die Bypassgasleitung 6 berechnet.

Aus dem ebenfalls mit dem mikrothermischen Sensor gemessenen Flussfaktor wird das Verhältnis zwischen Wärmekapazität und Wärmeleitfähigkeit des Gases oder Gasgemisches und, mit der bereits bekannten Wärmeleitfähigkeit, der Wert der Wärmekapazität berechnet. Schallgeschwindigkeit, Wärmeleitfähigkeit und Wärmekapazität werden anschliessend zur Korrelation der spezifischen Grössen für die Gasbeschaffenheit verwendet.

Da sich die oben beschriebenen Messungen und Berechnungen auf die Bypassgasleitung beziehen, wird auf das Teilungsverhältnis des Massenflusses zwischen Haupt- und Bypassgasleitung zurückgegriffen, um den Massenstrom in der Hauptgasleitung zu berechnen. Das Teilungsverhältnis kann beispielsweise im Voraus in einer Kalibriermessung mit bekannten Gasen bestimmt werden. Falls als spezifische Grösse für die Gasbeschaffenheit der Brennwert bestimmt wurde, liefert die Multiplikation des Massenstroms in der Hauptgasleitung mit dem Brennwert zusätzlich den Energieverbrauch.

Das Verfahren und die Messvorrichtung gemäss vorliegender Erfindung sowie die oben beschriebenen Ausführungsformen und -varianten zur Bestimmung von spezifischen Grössen für die Gasbeschaffenheit und/oder des Energieverbrauchs können in Hoch- und Niederdruckgasnetzen verwendet werden und ermöglichen dank der Korrelation aus den drei unabhängigen Variablen Schallgeschwindigkeit, Wärmeleitfähigkeit und Wärmekapazität eine vergleichsweise hohe Genauigkeit bei der Bestimmung der genannten Grössen.

**Patentansprüche**

1. Verfahren zur Bestimmung von spezifischen Grössen für die Gasbeschaffenheit, in welchem

   - ein Gas oder Gasgemisch durch einen Ultraschalldurchflusssensor (4) und über einen mikrothermischen Sensor (7) fliesst, und Temperatur (T) und Druck (p) des Gases oder Gasgemisches erfasst werden;
   - mit dem Ultraschalldurchflusssensor (4) sowohl die Fliessgeschwindigkeit ($v_x$) bzw. der Volumenfluss als auch die Schallgeschwindigkeit ($c_s$) des Gases oder Gasgemisches bestimmt werden;
   - aus der Schallgeschwindigkeit ($c_s$) die Dichte des Gases oder Gasgemisches korreliert wird;
   - die Dichteinformation, zusammen mit der Fliessgeschwindigkeit, zur Berechnung des Massenflusses verwendet werden;
   - mit Hilfe des mikrothermischen Sensors (7) die Wärmeleitfähigkeit ($\lambda$) des Gases oder Gasgemisches bei einer oder mehreren Temperaturen bestimmt wird;
   - aus dem Durchflusssignal des mikrothermischen Sensors (7) ein Flussfaktor ($\varphi$) berechnet wird, um aus demselben, zusammen mit der Information des Massenflusses und der Wärmeleitfähigkeit, die Wärmekapazität ($c_p$) oder eine von der Wärmekapazität abhängige Grösse des Gases oder Gasgemisches zu bestimmen;
   - schliesslich die Schallgeschwindigkeit, die Wärmeleitfähigkeit bei einer oder mehreren Temperaturen und entweder die Wärmekapazität oder die von der Wärmekapazität abhängige Grösse zur Korrelation von spezifischen Grössen für die Gasbeschaffenheit, insbesondere des Brennwerts, verwendet werden.

2. Verfahren nach Anspruch 1, wobei die mit dem Ultraschalldurchflusssensor bestimmte Schallgeschwindigkeit ($c_s$) auf die Schallgeschwindigkeit bei Normtemperatur ($T_{norm}$) umgerechnet wird.

3. Verfahren nach einem der Ansprüche 1 oder 2, wobei die mit Hilfe des mikrothermischen Sensors bestimmte Wärmeleitfähigkeit ($\lambda$) bei einer oder mehreren Temperaturen, zusammen mit der Schallgeschwindigkeit, zur genaueren Korrelation der Dichte verwendet werden.

4. Verfahren nach einem der Ansprüche 2 oder 3, wobei die mittels Korrelation ermittelte Dichte die Normdichte ist, und/oder wobei die mittels Korrelation ermittelte Dichte mit der Temperatur (T) und dem Druck (p) des Gases oder Gasgemisches auf die Dichte ($\rho$) bei Betriebsbedingungen umgerechnet wird.

5. Verfahren nach einem der Ansprüche 1 bis 4, wobei die Schallgeschwindigkeit, die Wärmeleitfähigkeit bei einer oder mehreren Temperaturen und entweder die Wärmekapazität oder die von der Wärmekapazität abhängige Grösse zur Korrelation des Brennwerts oder Wobbe-Index (W) oder Z-Faktors oder der kinematischen Viskosität verwendet werden.

6. Verfahren nach Anspruch 5, wobei aus dem Brennwert, zusammen mit dem Volumen- oder Massenfluss, der Energieverbrauch ($\Phi_{EN}$) errechnet wird.

7. Messvorrichtung zur Bestimmung von spezifischen Grössen für die Gasbeschaffenheit und/oder des Energieverbrauchs mit einem Drucksensor (8) zur Messung des Drucks, einem Temperatursensor (9) zur Messung der Temperatur und einem mikrothermischen Sensor (7) zur Messung der Wärmeleitfähigkeit und entweder der Wärmekapazität oder einer von der Wärmekapazität abhängigen Grösse des Gases oder Gasgemisches, **dadurch gekennzeichnet, dass** die Messvorrichtung (11) eine Auswerteeinheit (10), die zur Ausführung eines Verfahrens gemäss einem der Ansprüche 1 bis 6 eingerichtet ist, und einen Ultraschalldurchflusssensor (4) zur Messung von Schall- und Fliessgeschwindigkeit umfasst.

8.  Messvorrichtung gemäss Anspruch 7, wobei der Ultraschalldurchflusssensor (4) und der mikrothermische Sensor (7) in einer Gasleitung (1') anordnenbar und mit demselben Massenstrom beaufschlagbar sind.

9.  Messvorrichtung gemäss Anspruch 7, wobei der Ultraschalldurchflusssensor (4) in einer Hauptgasleitung (1) und der mikrothermische Sensor (7) in einer Bypassgasleitung (6) zur Hauptgasleitung anordnenbar sind, und in der Hauptgasleitung ein Druckabfall erzeugendes Element (5) vorgesehen ist, um in der Bypassgasleitung einen Massenstrom zu erwirken.

10. Messvorrichtung nach einem der Ansprüche 8 oder 9, wobei der Ultraschalldurchflusssensor (4) nicht invasiv auf die Gasleitung (1') oder Hauptgasleitung (1) aufsetzbar ist.

11. Messvorrichtung gemäss Anspruch 7, wobei der Ultraschalldurchflusssensor (4) und der mikrothermische Sensor (7) in einer Bypassgasleitung (6) zu einer Hauptgasleitung (1) anordnenbar sind, und in der Hauptgasleitung ein Druckabfall erzeugendes Element (5) vorgesehen ist, um in der Bypassgasleitung einen Massenstrom zu erwirken.

12. Messvorrichtung nach einem der Ansprüche 9 bis 11, wobei das Teilungsverhältnis (3) zwischen Massenfluss in der Bypass- (6) und in der Hauptgasleitung (1) im Voraus durch Kalibrieren mit einem bekannten Gas bestimmt worden ist.

13. Messvorrichtung nach einem der Ansprüche 7 bis 12, wobei die Messvorrichtung zusätzlich einen Abschnitt der Gasleitung (1') oder Hauptgasleitung (1) und/oder eine Bypassgasleitung (6), in denen mindestens einer der Sensoren (4, 7, 8, 9) der Messvorrichtung (11) angeordnet ist, und/oder ein Druckabfall erzeugendes Element (5) in der Hauptgasleitung (1) umfasst.

14. Messvorrichtung nach einem der Ansprüche 7 bis 13, wobei die Auswerteeinheit (10), zusammen mit der restlichen Messvorrichtung, eine Baueinheit bildet, oder wobei die Messvorrichtung ohne die Auswerteeinheit (10) eine Baueinheit bildet, und die Auswerteeinheit in einer separaten oder übergeordneten Recheneinheit ausgebildet ist.

**Claims**

1.  A method for determining specific quantities for the gas quality, in which

    - a gas or gas mixture flows through an ultrasonic flow sensor (4) and over a microthermal sensor (7), and the temperature (T) and the pressure (p) of the gas or gas mixture are detected;
    - the flow velocity ($v_x$) or volumetric flow as well as the sound velocity ($c_s$) of the gas or gas mixture are determined by the ultrasonic flow sensor (4);
    - the density of the gas or gas mixture is correlated from the sound velocity ($c_s$);
    - the density information, together with the flow velocity, are used for calculating the mass flow;
    - the thermal conductivity ($\lambda$) of the gas or gas mixture at one or several temperatures is determined with the microthermal sensor (7);
    - a flow factor ($\varphi$) is calculated from the flow signal of the microthermal sensor (7) in order to determine therefrom, together with the information of the mass flow and thermal conductivity, the thermal capacity ($c_p$) or a quantity of the gas or gas mixture dependent on the thermal capacity;
    - and finally the sound velocity, the thermal conductivity at one or several temperatures and either the thermal capacity or the quantity depending on the thermal capacity are used for correlation of specific quantities for the gas quality, in particular of the calorific value.

2.  A method according to claim 1, wherein the sound velocity ($c_s$) determined by the ultrasonic flow sensor is converted to the sound velocity at standard temperature ($T_{norm}$).

3.  A method according to one of the claims 1 or 2, wherein the thermal conductivity ($\lambda$) determined with the microthermal sensor at one or several temperatures is used, together with the sound velocity, for the preciser correlation of the density.

4. A method according to one of the claims 2 or 3, wherein the density determined by means of correlation is the standard density, and/or wherein the density determined by means of correlation is converted with the temperature (T) and the pressure (p) of the gas or gas mixture to the density ($\rho$) under operating conditions.

5. A method according to one of the claims 1 to 4, wherein the sound velocity, the thermal conductivity at one or several temperatures, and either the thermal capacity or the quantity depending on thermal capacity are used for correlation of the calorific value or Wobbe index (W) or Z factor or the kinematic viscosity.

6. A method according to claim 5, wherein the energy consumption ($\Phi_{EN}$) is calculated from the calorific value, together with the volumetric or mass flow.

7. A measuring apparatus for determining specific quantities for the gas quality and/or the energy consumption, comprising a pressure sensor (8) for measuring the pressure, a temperature sensor (9) for measuring the temperature, and a microthermal sensor (7) for measuring the thermal conductivity and either the thermal capacity or a quantity of the gas or gas mixture dependent on the thermal capacity, **characterised in that** the measuring apparatus comprises an evaluation unit (10) which is set up for carrying out a method according to one of the claims 1 to 6, and an ultrasonic flow sensor (4) for measuring the sound and flow velocity.

8. A measuring apparatus according to claim 7, wherein the ultrasonic flow sensor (4) and the microthermal sensor (7) are arrangeable in a gas line (1) and can be supplied with the same mass flow.

9. A measuring apparatus according to claim 7, wherein the ultrasonic flow sensor (4) is arrangeable in a main gas line (1) and the microthermal sensor (7) in a bypass gas line (6) to the main gas line, and an element (5) producing a pressure drop is provided in the main gas line in order to generate a mass flow in the bypass gas line.

10. A measuring apparatus according to one of the claims 8 or 9, wherein the ultrasonic flow sensor (4) is noninvasively placeable on the gas line or the main gas line (1).

11. A measuring apparatus according to claim 7, wherein the ultrasonic flow sensor (4) and the microthermal sensor (7) are arrangeable in a bypass gas line (6) to a main gas line (1), and an element (5) producing a pressure drop is provided in the main gas line in order to generate a mass flow in the bypass gas line.

12. A measuring apparatus according to one of the claims 9 to 11, wherein the splitting ratio between the mass flow in the bypass gas line and in the main gas line is determined in advance by calibration with a known gas.

13. A measuring apparatus according to one of the claims 7 to 12, wherein the measuring apparatus additionally comprises a section of the gas line (1') or of the main gas line (1) and/or a bypass gas line (6) in which at least one of the sensors (4, 7, 8, 9) of the measuring apparatus (11) is arranged, and/or an element (5) producing a pressure drop in the main gas line (1).

14. A measuring apparatus according to one of the claims 7 to 13, wherein the evaluation unit (10), together with the remaining measuring apparatus, forms a modular unit, or wherein the measuring apparatus forms a modular unit without the evaluation unit (10), and the evaluation unit is formed in a separate or higher-level computing unit.

**Revendications**

1. Procédé pour la détermination de grandeurs spécifiques de la nature d'un gaz, dans lequel

   - un gaz ou un mélange de gaz s'écoule à travers un débitmètre à ultrasons (4) et via un capteur microthermique (7), et la température (T) et la pression (p) du gaz ou du mélange de gaz sont détectées ;
   - le débitmètre à ultrasons (4) est utilisé pour déterminer à la fois la vitesse d'écoulement ($v_x$) ou le débit volumique et pour déterminer la vitesse du son ($c_s$) du gaz ou du mélange de gaz ;
   - la densité du gaz ou du mélange de gaz est corrélée avec la vitesse du son ($c_s$) ;
   - l'information de densité et la vitesse d'écoulement sont utilisées pour calculer le débit massique ;
   - la conductivité thermique ($\lambda$) du gaz ou du mélange de gaz à une ou plusieurs températures est déterminée à l'aide du capteur microthermique (7) ;
   - un facteur de débit ($\varphi$) est calculé à partir du signal de débit du capteur microthermique (7) afin de déterminer à partir de celui-ci, ainsi que de l'information du débit massique et de la conductivité thermique, la capacité thermique ($c_p$) ou une grandeur du gaz ou du mélange de gaz dépendante de la capacité thermique;

- enfin, la vitesse du son, la conductivité thermique à une ou plusieurs températures et soit la capacité thermique, soit la grandeur dépendante de la capacité thermique sont utilisées pour la corrélation de grandeurs spécifiques de la nature du gaz, en particulier du pouvoir calorifique.

2. Procédé selon la revendication 1, dans lequel la vitesse du son ($c_s$) déterminée avec le débitmètre à ultrasons est convertie en vitesse du son à la température normale ($T_{norm}$).

3. Procédé selon l'une des revendications 1 ou 2, dans lequel la conductivité thermique ($\lambda$) à une ou plusieurs températures déterminée à l'aide du capteur microthermique, ainsi que la vitesse du son, sont utilisées pour une corrélation plus précise de la densité.

4. Procédé selon l'une des revendications 2 ou 3, dans lequel la densité déterminée par corrélation est la densité normale et/ou dans lequel la densité déterminée par corrélation est convertie avec la température (T) et la pression (p) du gaz ou du mélange de gaz en la densité ($\rho$) dans les conditions opératoires.

5. Procédé selon l'une des revendications 1 à 4, dans lequel la vitesse du son, la conductivité thermique à une ou plusieurs températures et soit la capacité thermique, soit la grandeur dépendante de la capacité thermique sont utilisées pour corréler le pouvoir calorifique, l'indice de Wobbe (W), le facteur Z ou la viscosité cinématique.

6. Procédé selon la revendication 5, dans lequel la consommation d'énergie ($\Phi_{EN}$) est calculée à partir du pouvoir calorifique et du débit volumique ou massique.

7. Dispositif de mesure pour déterminer des grandeurs spécifiques de la nature d'un gaz et/ou la consommation d'énergie, comprenant un capteur de pression (8) pour mesurer la pression, un capteur de température (9) pour mesurer la température et un capteur microthermique (7) pour mesurer la conductivité thermique et soit la capacité thermique, soit une grandeur du gaz ou du mélange de gaz dépendante de la capacité thermique, **caractérisé en ce que** le dispositif de mesure (11) comprend une unité d'évaluation (10) qui est conçue pour mettre en œuvre un procédé selon l'une des revendications 1 à 6 et un débitmètre à ultrasons (4) pour mesurer la vitesse du son et la vitesse d'écoulement.

8. Dispositif de mesure selon la revendication 7, dans lequel le débitmètre à ultrasons (4) et le capteur microthermique (7) peuvent être disposés dans une conduite de gaz (1') et peuvent être exposés au même débit massique.

9. Dispositif de mesure selon la revendication 7, dans lequel le débitmètre à ultrasons (4) peut être disposé dans une conduite de gaz principale (1) et le capteur microthermique (7) dans une conduite de gaz de dérivation (6) de la conduite de gaz principale, et un élément produisant une baisse de pression (5) est prévu dans la conduite de gaz principale afin de produire un débit massique dans la conduite de gaz de dérivation.

10. Dispositif de mesure selon l'une des revendications 8 ou 9, dans lequel le débitmètre à ultrasons (4) peut être placé de manière non invasive sur la conduite de gaz (1') ou la conduite de gaz principale (1).

11. Dispositif de mesure selon la revendication 7, dans lequel le débitmètre à ultrasons (4) et le capteur microthermique (7) peuvent être disposés dans une conduite de gaz de dérivation (6) d'une conduite de gaz principale (1), et un élément produisant une baisse de pression (5) est prévu dans la conduite de gaz principale afin de produire un débit massique dans la conduite de gaz de dérivation.

12. Dispositif de mesure selon l'une des revendications 9 à 11, dans lequel le rapport de division (3) entre le débit massique dans la conduite de gaz de dérivation (6) et dans la conduite de gaz principale (1) a été préalablement déterminé par étalonnage avec un gaz connu.

13. Dispositif de mesure selon l'une des revendications 7 à 12, lequel dispositif de mesure comprend en outre une partie de la conduite de gaz (1') ou de la conduite de gaz principale (1) et/ou une conduite de gaz de dérivation (6), dans lesquelles est disposé au moins l'un des capteurs (4, 7, 8, 9) du dispositif de mesure (11), et/ou un élément produisant une baisse de pression (5) dans la conduite de gaz principale (1).

14. Dispositif de mesure selon l'une des revendications 7 à 13, dans lequel l'unité d'évaluation (10) forme avec le reste du dispositif de mesure une unité modulaire ou dans lequel le dispositif de mesure sans l'unité d'évaluation (10) forme une unité modulaire et l'unité d'évaluation est réalisée dans une unité de calcul séparée ou supérieure.

**Fig. 1a**

**Fig. 1b**

$$t_{12} = \frac{L}{c_s + v_x \cdot \cos\alpha};$$

$$t_{21} = \frac{L}{c_s - v_x \cdot \cos\alpha}$$

$$v_x = \frac{L}{2 \cdot \cos\alpha \cdot t_{12} \cdot t_{21}}(t_{21} - t_{12});$$

$$c_s = \frac{L}{2 \cdot t_{12} \cdot t_{21}}(t_{21} + t_{12})$$

**Fig. 2a**

**Fig. 2b**

**Fig. 3a**

**Fig. 3b**

**Fig. 3c**

**Fig. 4**

# Fig. 5

**Fig. 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0715169 A1
- EP 2015056 A1
- WO 2004008136 A1
- WO 0240992 A1
- EP 14001767 A
- EP 2574918 A1

**In der Beschreibung aufgeführte Nicht-Patentliteratur**

- **D. MATTER ; B. KRAMER ; T. KLEINER ; B. SABBATTINI ; T. SUTER.** Mikroelektronischer Haushaltsgaszähler mit neuer Technologie. *Technisches Messen,* 2004, vol. 71 (3), 137-146
- **KERSON HUANG.** Statistical Mechanics. John Wiley & Sons, 1987
- **L.C. LYNNWORTHA ; YI LIUB.** Ultrasonic flowmeters: Half-century progress report, 1955-2005. *Ultrasonics,* 2006, vol. 44, e1371-e1378